# EUROPEAN PATENT SPECIFICATION

(11) **EP 1 734 959 B1**
(45) Date of publication and mention of the grant of the patent: **28.10.2009**
(21) Application number: 05732721.5
(22) Date of filing: 01.04.2005
(51) Int. Cl.: A61K 31/4439, A61P 25/18, A61P 3/10

(54) **COMPOUNDS FOR TREATING SCHIZOPHRENIA AND/OR GLUCOREGULATORY ABNORMALITIES**
VERBINDUNGEN ZUR BEHANDLUNG VON SCHIZOPHRENIE UND/ODER GLUCOREGULATORISCHE AUFFÄLLIGKEITEN
COMPOSÉS POUR LE TRAITEMENT DE LA SCHIZOPHRENIE ET / OU DES ANOMALIES DE LA GLUCOREGULATION

(30) Priority: 01.04.2004 US 558451 P
(43) Date of publication of application: 27.12.2006
(73) Proprietor: Aventis Pharmaceuticals Inc., Bridgewater, New Jersey 08807 (US)
(72) Inventor: SMITH, Craig, P., Hillsborough, NJ 08844 (US); RAMPE, David, E., Bernardsville, NJ 07924 (US); BOROWSKY, Beth, Flemington, NJ 08822 (US); KONGSAMUT, Sathapana, Madison, NJ 07940 (US)
(74) Representative: Wein, Elmar Michael
(86) International application number: PCT/US2005/011107
(87) International publication number: WO 2005/097122

(56) References cited:
- EP-A- 0 287 982
- WO-A-97/04777
- WO-A-02/064126
- US-A- 5 356 910
- SMITH C P ET AL: "N-(N-PROPYL)-N-(3-FLUORO-4-PYRIDINYL)-1H- 3-METHYLINDOL-1-AMINE HYDROCHLORIDE (HP 184): IN VITRO SPONTANEOUS RELEASE OF ACETYLCHOLINE AND NOREPINEPHRINE" DRUG DEVELOPMENT RESEARCH, NEW YORK, NY, US, vol. 30, no. 4, December 1993 (1993-12), pages 203-212, XP001096106 ISSN: 0272-4391

## Description

### BACKGROUND OF THE INVENTION

Schizophrenia is present in about 1% of the population worldwide. It is estimated that about 2.8 million people in the US alone have the disorder. In spite of antipsychotic drugs 30 - 40% of patients remain treatment resistant. A similar number remain considerably symptomatic with residual negative (see below) and psychotic (eg, hallucinations and delusions) symptoms. On the other hand, psychosis in schizophrenia is a remitting relapsing disorder with deteriorating forms. 60 to 80% of patients will relapse or worsen in 1 to 2 years even on maintenance antipsychotic treatment. Although psychotic symptoms are the signal for antipsychotic drug treatment, other clinical domains of schizophrenia are more socially and functionally disabling. Only 7% of patients will be fully employed, while only one in five will be working at some time in their lives.

Antipsychotic drugs do not satisfactorily treat cognitive impairments or negative symptoms of schizophrenia, including social competence and problem solving (eg, Bellack et al, 2004). Cognitive impairments and negative symptoms are considered to be the core of the disorder. Both domains are believed the major cause of deterioration of social and occupational functioning, caretaker burden, and increased risk of psychotic relapse (Verdoux et al., 2002; van Kammen et al, unpublished data, 1996).

Negative symptoms such as flat affect, social and emotional withdrawal, poverty of content of speech, lack of motivation, anhedonia (inability to experience pleasure), apathy, motor retardation and lack of personal hygiene are major disabling symptoms of the disorder, that do not respond well to antipsychotics. At any given time, negative symptoms may be present in 60% of patients. Negative symptoms can be present before the onset of the illness and worsen over time. Although they tend to be stable, they do improve somewhat with improvement in psychosis. Antipsychotic drugs may induce negative symptoms (eg, flatness of affect, emotional withdrawal, motor retardation) through dopamine receptor blockade (extrapyramidal side effects or EPS). Negative symptoms, induced by dopamine receptor blockade, psychosis or depression, are called secondary (non-enduring) negative symptoms. Those negative symptoms may respond to switching to serotonin-dopamine antagonists (SDAs) with fewer EPS, lowering the dose of antipsychotics, to prescribing anticholinergic agents or antidepressants. However, anticholinergic agents induce their own (recent) memory impairments. Enduring primary negative symptoms are also called the deficit syndrome. Those are unresponsive to our present day antipsychotics. Increased negative symptoms are associated with a slower and poorer antipsychotic response, while more negative symptoms indicate a greater cognitive impairment.

Cognitive abnormalities are pervasively present in a broad spectrum in patients with schizophrenia. Approximately 85% of patients with schizophrenia exhibit some degree of cognitive impairment (Palmer et al., 1999; McGurk and Meltzer, 2000; Meltzer and McGurk, 1999). The remaining 15% of patients, who score within the normal range on some cognitive tests often perform at levels below those of their siblings and parents, indicating that these patients may not be functioning at their full potential. They may still have abnormal regional brain activation on fMRI during cognitive testing (activation of alternative regions or increased activity). Sustained attention is almost always impaired (Goldstein et al, 1999). In spite of a variable cognitive impairment across patients with schizophrenia, basically all patients have some form of cognitive impairment. These deficits are present in antipsychoticnaïve patients with first-episodes schizophrenia as well in medicated chronic patients. Patients with higher IQ scores may be better able to avoid hospitalization (greater compensatory reserve?), but can be as psychotic as other patients.

The following cognitive categories are impaired in schizophrenia: sustained attention, working memory (eg, executive function), verbal learning and memory, visual learning and memory (visual-spatial), speed of processing and word fluency, reasoning and problem solving, and social cognition. Although it makes intuitive sense that psychosis should interfere with cognitive tests, positive symptoms (eg, hallucinations, delusions) do not correlate significantly with cognitive impairments or outcome (Seaton et al, 1999) or functional outcome over time. Thought disorder, negative symptoms and cognitive impairments do (Green, 1996, 1999).

Neuropsychological impairments are associated with negative symptoms (eg, psychomotor poverty). They include a) impairments in working memory, b) a weakening of the influences of stored memories of previous input on current perception, with links to impaired latent inhibition and c) abnormal lateralization of cognitive functioning, emphasizing either left or right cerebral dysfunction.

In general, most patients show a relative, stable cognitive impairment during adulthood with a relatively small annual decline. On the Mini-Mental Status Exam (MMSE) patients with schizophrenia decline 2-3 points over 50 years, in contrast with patients with Alzheimer Disorder who loose 1.5 points per year. In addition to cognitive impairment prior to the appearance of psychosis, further deterioration may occur with the first one or two psychotic episodes. Particularly, impairment in executive functioning, semantic memory and speeded motor performance tends to worsen over time. Some patients improve slightly after the initial worsening associated with early psychotic episodes, but this improvement is not observed in all tests. Executive functioning is particularly unresponsive to antipsychotic treatment. An earlier the age of onset and more negative symptoms are associated with a greater cognitive impairment (dementia praecox). Although studies with atypical or SDA antipsychotics indicate that risperidone, olanzapine, ziprasidone or quetiapine significantly may improve cognition in schizophrenia, the response to these agents is rather small, inconsistent or insufficient. Executive functioning (working memory) does not improve with the SDAs. Clozapine has been shown to have the strongest positive effect, however the anticholinergic side effects interfere with recent memory.

In addition, recent evidence suggests that cognitive impairment is a much better predictor of poor social functioning and employment in patients with schizophrenia than hallucinations or delusions (Green, 1996; Green, 1999). Particularly, secondary (storage) memory may be a consistent predictor of work and social function.

Multiple hypotheses have been put forward to explain the underlying mechanisms of the cognitive deficits. Abnormal connectivity (eg, decreased neuronal arborization, increased synaptic turnover, increased white matter neurons, white matter impairments --particularly in uncinate fasciculus, longitudinal fasciculus, corpus callosum, and in the prefrontal, parietal and temporal lobe), altered neurotransmitter activity (eg, decreased prefrontal dopamine, decreased glutamate activity, decreased acetylcholine, hypercortisolemia (Walker and Diforio, 1997;Walder et al, 2000; Newcomer et al, 1991; Altamura et al, 1991), subsensitive □7nicotinic receptor stimulation in the temporal lobe, increased COMT activity in the dorsolateral prefrontal cortex (DLPFC), altered alpha receptor activity and pharmacological mechanisms (eg, D₁ receptor blockade in PFC, anticholinergic mechanisms, extrapyramidal side effects (Cassens et a. 1990)). Hypercortisolemia may play a major role in the cognitive deficit on different levels. It is associated with increased glutamate activity (eg, effects on gray and white matter) and stimulation of IL-6, IL1B and IL2 (Zhang et al, in press). On an aside, it is of interest that as a group, female patients with schizophrenia score better than their male counterparts on cognitive tests, including responding better to cognitive therapy. This difference cannot be explained on the basis of estrogens.

*Gray matter.* Decreased brain volume has been noted in schizophrenia since 1976, when Eve Johnstone reported the first brain CT scan study in schizophrenia. Subsequent CT brain scan studies confirmed this finding emphasizing cortical atrophy and wider lateral ventricles (for review see Goetz and van Kammen, 1984). Initial PET scan studies identified a hypofrontality (Buchsbaum et al, 1982), which later was clarified with fMRI studies indicating a decreased activation in the dorsolateral prefrontal cortex (DLPC) in schizophrenia (Berman and Weinberger) as well as decreased neuropil from autopsy studies (eg, Selemon et al;). This decreased in gray matter without cell loss has been explained by a decrease in synapse formation, dendritic spines and arborization. During normal development neuronal pruning and an increase in synaptogenesis occur. Neuronal pruning takes place particularly during adolescence. By the age of 30, we have already lost 50% of the neurons we were born with. In the normal brain synaptogenesis and synaptic reduction takes place concomitantly. Long-term memory (learning) requires neuronal growth and synaptogenesis.

Studies indicating altered neuronal migration patterns, with increased neurons in white matter areas, particularly in the temporal cortex, (eg, Jakob and Beckman, 1986) have been reported, albeit not consistently. Such findings suggest disturbances in neurodevelopment. These studies combined with epidemiological studies of increased incidence of severe stress or viral infections, or hemorrhage/ischemia in utero in the 2^{nd} or 3^{rd} trimester and perinatally (in the presence of genetic abnormalities) let to the formulation of schizophrenia as a disorder of neurodevelopmental, ie interference with normal brain development, which expresses it self during or after adolescence (Weinberger, 1987; Murray, 1987).

fMRI studies with working memory tasks identified decreased DLPFC activity. This is in addition to decreased volume in the DLPFC (see above). In healthy controls stimulation of the DLPFC prefrontal cortex leads to activation of the temporal lobe, but not in schizophrenia. Extensive psychophysiology, MRI and PET studies identified altered connectivity and information processing in schizophrenia brains. In addition to hypoactivity also increased activity was noted in other brain areas not activated in healthy controls during certain cognitive tasks (McCarley et al; Liddle et al). With improved brain autopsy and imaging technology, identification of decreased neuropil (eg, shrinkage of neuronal mass, decreased arborization and altered synaptic proteins, decreased synaptic formation), but little evidence of actual neuronal loss, except perhaps of some cortical GABAergic intemeurons (Lewis; Benes et al, 1999) have been identified. Gray matter decreases have been associated with positive and negative symptoms, as well as cognitive deficits and poor functional outcome in schizophrenia.

*White matter.* Over 40% of brain volume is white matter. White matter develops over time and continues to expand into the 5^{th} decade, partially making up for space provided by neuronal pruning and neuronal loss (see gray matter). While cortical association areas complete their myelination after the motor areas, the prefrontal cortex does not reach full myelination until puberty. The dopamine system is among the latest to be myelinated. For normal myelination to occur normal conduction is required. Myelination is the ultimate neuroprotectant, assuring optimal neurotransmission. Impaired myelination will cause decreased conduction velocity, and expose neurons to increased metabolic toxins. This means a decrease in neuronal functioning, brain reserve and resilience. However, myelination is not an indispensable property of functional axons. Axons can still function without myelination. Myelination is just one criterion of neural maturation.

Since 1998 decreases in white matter volume have been reported in the PFC, temporal lobe, perigenual cingulate gyrus, corpus callosum, thalamus, and parietal lobe of patients with schizophrenia (for review see Davis et al., 2002 and Bartzokis, 2002). In addition, autopsy and genetic studies have reported impaired myelination in schizophrenia (see below). Alterations in white matter have been associated with impaired cognition and increased negative symptoms (Kubicki et al, 2003; Lim et al; Wolkin et al. 1998; Foong et al, 2000, 2001). Because the white matter volume deficit compared to healthy controls increases with age, a diminished or halted myelination process has been suggested (Bartzokis et al. 2003; Andreassen). The following observations have also been reported:
- Arrest of normal myelination increases on MRIs (Andreasen et al): a decrease in relative brain growth, a decrease in gray/white matter ratios - greater decline between two MRIs over time, associated with more negative symptoms.
- Abnormalities in oligodendroglia:
   Decreased number of oligodendrocytes in PFC (area 9): 28% in layer III and 27% in layer V and VI (Hof et al, 2003); in the PFC and caudate (Miyakawa et al, 1972; Deicken et al, 1994; Keshavan et al, 1998).
- Electron Microscopy: abnormalities in ultrastructure of myelin sheath lamellae in frontal cortex biopsy (Orlovskaya et al, 2000; Uranova et al, 2002) and post-mortem brains (Miyakawa et al, 1972; Orlovskaya et al, 2000); concentric inclusion bodies between lamellae, loss of sheath compactness, swelling (Torrey et al, 2000; Cotter et al, 2000), signs of apoptosis and necrosis and apoptotic cell death of oligodendrocytes and focal demyelination as in MS, an increase in heterochromatin in tracts in caudate and PFC (Uranova et al, 2000).
- Abnormalities in myelin: Magnitization Transfer Ration (MTR) or Diffusion Tension Imaging (DTI) measures anoisotropy in white matter tracts. Such MRI studies have indicated impaired lamination (myelination) in major tracts in schizophrenia and significant correlations with cognitive testing and negative symptoms. (Foong et al, 2000,2002; Buchsbaum et al 1998; Lim et al 1999; Shihabuddin et al 2000; Agartz et al 2001; Kubicki et al 2002, 2003; Wolkin et al 1998, 2003). Relationships of MTR or DTI with cognitive scores are not present in healthy controls but in patients with schziophrenia.

Several groups have shown that many of the genes involved in myelination are abnormal in schizophrenia (Davis et al, 2002; Tkachev et al, 2003; Hakak et al., 2001; Pongrac et al., 2002; Mimmack et al., 2002; Stefansson et al., 2002). Although these research groups have replicated in general their myelin related gene abnormalities, not all groups have identified the same genes or observed them in the same direction. Particularly younger patients show an increase in myelin related gene activity, while older Kraepelinian patients show a decrease in those genes (Davis et al, 2002; Hakak et al., 2001). Presumably, the failed attempts to jack up gene activity to overcome the impairments in myelination finally run out later in life, with the associated rapid deterioration in those older patients.

For years, disconnectivity (or dysfunctional brain architecture) hypotheses of schizophrenia (McGuire and Frith, 1996; Friston, 1998;) have been at pains to explain the information processing difficulties in schizophrenia with gray matter abnormalities alone without invoking white matter abnormalities. Only recently has a wealth of these white matter studies indicated that indeed white matter abnormalities are present in schizophrenia, and in particular in the white matter tracts. The neurodevelopmental hypothesis based upon gray matter changes explains mainly the onset of schizophrenia into late adolescence or early adulthood. The peak onset of schizophrenia occurs in young adulthood but onset of schizophrenia can occur as late as in the fifth decade. The recent reports of white matter changes into the fifth decade of life have made the neurodevelopmental and disconnection hypotheses more consistent with the clinical data, ie, an age of onset into the 50's.

Brain imaging studies have provided evidence of lower volume, metabolic activity or brain blood flow in specific brain regions (eg, DLPC), suggesting localized brain lesion(s) in schizophrenia (Selemon; Goldman-Rakic; Benes). In spite of the evidence from human and animal lesion studies, that specific brain lesions may cause memory and other problems in cognition, there is no evidence that such localized lesions are involved in schizophrenia. Brain autopsy studies going back to the end of the 19^{th} century have failed to identify such specific brain lesions, even with the improved methodologies of recent studies. Interestingly, psychosis can occur in patients with white matter disorders such as multiple sclerosis (MS), metachromatic leukodystrophy (MLD) and traumatic brain injury (TBI), particularly when lesions occur in the frontal cortex tracts. In MS, the white matter disorder par excellence, psychosis is associated with white matter lesions in the frontal cortex, while MLD occurring in adolescence or early adulthood may present it self as schizophrenia. TBI is sometimes associated with psychosis when frontal white matter tracts are interrupted (shearing). Schizophrenia, without such identifiable lesions, is therefore associated with different white matter disorder than MS, MLD or TBI.

A more compelling interpretation of the data is the conventional hypothesis of functional lesions in schizophrenia. This functional lesion may well be in the impaired white matter tracts such as uncinate, longitudinal fasciculus, temporo-parietal or fronto-parietal tracts (ie, decreased anisotropy, with DTI or MRT MRI) or in the synaptic strength of the neurons (Kubicki et al, 2003; Foong et al; Lim et al; Buchsbaum et al, 1998). Connectivity is the ability of different brain regions to communicate with each other effectively. Several investigators have shown that synchrony or communication between different brain areas is altered in schizophrenia (Cleghorn et al, 1992; Liddle at al; Woodruff et al, 1997). In other words, the different brain areas, which need to work together in the many circuits in the normal functioning brain, are functionally disconnected. These disconnectivity hypotheses are based on weakened or altered neuronal transmission between different neuronal areas leading to decreased or altered activation of the necessary neuronal circuits, without evidence of actual cell lesions or neuronal loss. Altered synaptic strength, impaired myelination or both may cause this disconnectivity, although other causes cannot be ruled out. Questions have been raised whether these white matter abnormalities in schizophrenia are based upon a neurodevelopmental disturbance, as is hypothesized for the gray matter changes. Such alterations may decrease long-term memory, working memory, processing speed and word fluency, as well as negative symptoms.

For efficient functioning of the brain groups of neurons or networks need to fire at the same time (synchronicity). Alterations in this synchronicity (simultaneously firing) indicate inefficient or ineffective processing of information. Such responses can be used to identify changes in conduction velocity (ie, latency of amplitude, amplitude). Averaged evoked response (AER) or Event-Related potentials (ERP) are EEG wave amplitudes to a specified repeated stimulus. The amplitude of such brain waves will be significantly larger than when neurons fire randomly. A larger amplitude suggests more efficient processing of the stimulus and greater synchrony. Impaired conduction velocity, either through white or gray matter alterations may explain the many abnormal (psycho-) physiological tests results in schizophrenia: decreased reaction time, increased latency and decreased amplitude on AER (eg, p300, N400, p50), decreased mismatch negativity (MMN), early response of anti-sacchadic eye tracking (SPEM), decreased olfactory function, as well as impaired cognition (eg, decreased speed of processing, impaired backward masking, verbal fluency), abnormal fMRI activation(eg, hypo or hyperactivity, or activation of different regional areas in the brain), and the impaired social and functional outcome in schizophrenia.

In summary, negative symptoms (primary enduring symptoms or deficit syndrome), cognitive impairment, altered psychophysiology and brain-imaging responsiveness may all be expressions of an underlying hypoconnectivity or altered functional architecture of the brain: eg, decreased conduction velocity, secondary to impaired myelination and synaptic weakness. Negative symptoms, cognitive deficit, psychophysiological measures such as the AER p300 (latency and amplitude) and brain imaging eg, diffusion tensor imaging or magnetization transfer MRI may improve with improvement in conduction velocity and myelination.

Hyperglycemia and type 2 diabetes mellitus are more common in schizophrenia than in the general population. Glucoregulatory abnormalities have also been associated with the use of antipsychotic medications themselves. Arch Gen Psychiatry. 2002 Apr;59(4):337-45. Newer antipsychotics, so-called "atypical antipsychotics", induce minimal extrapyramidal side effects (EPS). The lack of EPS is believed to be due to the relatively greater affinity of these new antipsychotics for certain nondopaminergic receptors than their older, conventional counterparts. However, this polyreceptor affinity may be responsible for the development of metabolic side effects such as, for example, glucose intolerance, new-onset type 2 diabetes, diabetic ketoacidosis (DKA) and hypertriglyceridemia. H. Jin et al. Schizophrenia Research 2004.

Diabetes is a group of metabolic diseases with characteristic hyperglycemia associated with defects in insulin secretion, insulin action, or both. Diabetes is widely recognized as one of the leading causes of death and disability contributing to the deaths in the USA alone of more than 169,000 persons in 1992. Its toll increases every year.

There are several different types of diabetes. Type 2 (Non-Insulin dependent) diabetes may range from predominantly insulin resistance with relative insulin deficiency to a predominantly secretory defect with insulin resistance. Type 2 diabetes affects more than 15 million adult Americans and the prevalence is increasing. Persons with diabetes experience significant illness and even death from a variety of long term effects of elevated blood glucose levels. This is related to the damage of blood vessels in important organs such as the heart, eye, and kidney.

Although the precise mechanism of the antipsychotic-associated diabetes is unclear, it has been hypothesized that histaminic and possibly serotonergic antagonism induces weight gain. This antagonism is believed to cause changes in glucose regulation. Scientists also postulate that serotonin1A antagonism might decrease pancreatic beta-cell responsiveness, resulting in inappropriately low insulin and hyperglycemia. Biol Psychiatry. 1998 Oct 15;44(8):778-83.

In 2003, the FDA stated its position that all drugs for schizophrenia - known as atypical antipsychotics - should have labels warning of increased risk of diabetes. Thus, there is a high unmet medical need to find new antipsychotic therapeutics that have no adverse effects on glucose regulation. It is also clear that there is a need for improved antidiabetic agents to combat the diabetes epidemic.

Kv2.1 is a voltage-dependent K+ channel found throughout the CNS as well as in other tissues. Blockade of Kv2.1 channels has been linked to enhancement of glucose-stimulated insulin release. A putatively selective blocker of the Kv2.1 channel [bispidine derivative (C-1)] enhances glucose-stimulated insulin release (MacDonald et al, 2002). Kv2.1 channel blockade is believed to enhance the post-prandial insulin response (Roe et al, 1996; MacDonald et al, 2001; 2002).

Blockade of Kv1.3 channels has recently been shown to increase insulin sensitivity in mice (Xu et al, 2004). Indeed, Kv1.3 knock-out mice gain less weight than controls when fed high fat diets (Xu et al, 2003).

A compound which blocks either the Kv2.1 channel or the Kv1.3 channel, or both, may act on pancreatic β-cells to stimulate insulin release, especially glucose-stimulated insulin release thereby making it useful as an antidiabetic agent. Furthermore, a compound that additionally has the ability to enhance conduction velocity and repair white matter should provide a new and useful antipsychotic medication, i.e. one without the propensity to induce diabetes in the schizophrenic patient population.

### SUMMARY OF THE INVENTION

The instant application provides a compound of Formula I for use in enhancing glucose-stimulated insulin release in a patient in need thereof. The instant application also provides a compound of Formula I for use in treating schizophrenia and/or glucoregulatory abnormalities in a patient in need thereof.

### DETAILED DESCRIPTION OF THE INVENTION

United States Application Serial No. 10/076191 and WO 02/064126A disclose that the compounds of formula I provide a unique combination of blocking properties for both the potassium and sodium channels. This unique combination of blocking properties means that these compounds are useful as therapeutic agents for the treatment of demyelinating diseases or conditions such as multiple sclerosis, spinal cord injury, traumatic brain injury and stroke. The '191 application also discloses that the compounds are useful for stroke rehabilitation, the treatment of bladder irritation and dysfunction, the treatment of visceral, chemokine-induced pain (including arthritic pain) and neuropathic pain. 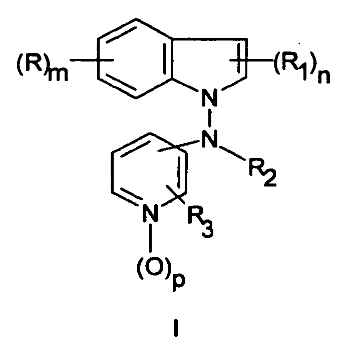 wherein
m is 0, 1 or 2;
n is 0, 1 or 2;
p is 0 or 1;
each R is independently hydrogen, halogen, trifluoromethyl, C₁-C₆alkyl, C₁-C₆alkoxy, benzyloxy, hydroxy, nitro or amino;
each R₁ is independently hydrogen, C₁-C₆alkyl, C₁-C₆alkenyl, C₁-C₆alkanoyl, halogen, cyano, -C(O)C₁-C₆alkyl, -C₁-C₆alkyleneCN, -C₁-C₆alkyleneNR'R" wherein R' and R" are each independently hydrogen or C₁-C₆alkyl, -C₁-C₆alkyleneOC(O)C₁-C₆alkyl, or -CH(OH)R₄ wherein R₄ is hydrogen or C₁-C₆alkyl;
R₂ is hydrogen, C₁-C₆alkyl optionally substituted with halogen, hydroxy or benzyloxy, C₁-C₆alkenyl, C₁-C₆alkynyl, -CO₂C₁-C₆alkyl, or -R₅-NR' R" wherein R₅ is C₁-C₆alkylene, C₁-C₆alkenylene or C₁-C₆alkynylene and R' and R" are each independently hydrogen, C₁-C₆alkyl or alternatively the group -NR'R" as a whole is 1-pyrrolidinyl; and R₃ is hydrogen, nitro, amino, halogen, C₁-C₆alkoxy, hydroxy or C₁-C₆alkyl.

Other references disclose the use of a compound of formula (I), including N-(4-Pyridyl)-N-propyl-1H-indol-1-amine and N-(3-fluoro-4-Pyridyl)-N-propyl-3-methyl-1H-indol-1-amine, to treat convulsions (WO 97/04777A); to treat obsessive compulsive disorder (US 5356910); to treat Alzheimer's disease (Drug Development Research, Vol. 30, No. 4, December 1993, pages 203-212); to enhance memory, as analgesic and as antidepressant (EP0287982A).

### Definitions:

1) Alkyl or alkylene - Unless otherwise stated or indicated, the term "Alkyl" or "alkylene" means a branched or straight chain alkyl or alkylene group, as is appropriate to the formula, specified by the amount of carbons in the alkyl, e.g., C₁-C₆ alkyl means a one, two, three, four, five or six carbon branched or straight chain alkyl or alkylene, as the case may be, or any ranges thereof, for example, C1-2, C1-3, C1-4, C1-5, C2-3, C2-4, C2-5, C2-C6, C3-C4, C3-5, C3-6, C4-5, C4-6, C5-6.
2) C₁-C₆alkoxy -Unless otherwise stated or indicated, the term C₁-C₆alkoxy denotes a straight or branched alkoxy group having from 1 to 6 carbon atoms. Examples of said include methoxy, ethoxy, n-proxy, isopropoxy, n-butoxy, iso-butoxy, sec-butoxy, t-butoxy and straight-and branched-chain pentoxy and hexoxy.
3) Halogen - Unless otherwise stated or indicated, the term halogen shall mean fluorine, chlorine, bromine or iodine.
4) C₁-C₆alkanoic acid - Unless otherwise stated or indicated, the term C₁-C₆alkanoic acid shall mean a carboxylic acid in which the carboxyl group is attached to hydrogen or an alkyl group of from 1 to 5 carbon atoms.
5) C₁-C₆alkanoyl - The term C₁-C₆alkanoyl shall mean a group obtained by removing a hydroxy group from the carboxyl group of a C₁-C₆alkanoic acid, and thus it includes for instance formyl, acetyl. The terms alkanoyl, alkenoyl and alkynoyl shall mean groups obtained by removing a hydroxy group from the carboxyl group of alkanoic acid, alkenoic acid and alkynoic acid, respectively. Thus, for instance, linoleyl group derived from linoleic acid is an example of the term alkenoyl as defined above.
6) "Pharmaceutically acceptable salts" means either an acid addition salt or a basic addition salt which is compatible with the treatment of patients for the intended use.
7) "Pharmaceutically acceptable acid addition salt" is any non-toxic organic or inorganic acid addition salt of the base compounds represented by Formula I or any of its intermediates. Illustrative inorganic acids which form suitable salts include hydrochloric, hydrobromic, sulfuric and phosphoric acid and acid metal salts such as sodium monohydrogen orthophosphate and potassium hydrogen sulfate. Illustrative organic acids which form suitable salts include the mono-, di- and tri-carboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicyclic, 2-phenoxybenzoic, p-toluenesulfonic acid and sulfonic acids such as methanesulfonic acid and 2-hydroxyethanesulfonic acid. Either the mono- or di-acid salts can be formed, and such salts can exist in either a hydrated, solvated or substantially anhydrous form. In general, the acid addition salts of these compounds are more soluble in water and various hydrophilic organic solvents and which in comparison to their free base forms, generally demonstrate higher melting points.
8) "Pharmaceutically acceptable basic addition salts" means non-toxic organic or inorganic basic addition salts of the compounds of Formula (I) or any of its intermediates. Examples are alkali metal or alkaline-earth metal hydroxides such as sodium, potassium, calcium, magnesium or barium hydroxides; ammonia, and aliphatic, alicyclic, or aromatic organic amines such as methylamine, trimethylamine and picoline. The selection criteria for the appropriate salt will be known to one skilled in the art.
9) "Stereoisomers" is a general term for all isomers of the individual molecules that differ only in the orientation of their atoms in space. It includes mirror image isomers (enantiomers), geometric (cis/trans) isomers, and isomers of compounds with more than one chiral center that are not mirror images of one another (diastereoisomers).
10) "Patient" means a warm blooded animal, such as for example rat, mice, dogs, cats, guinea pigs, and primates such as humans.
11) "Treat" or "treating" means to alleviate symptoms, eliminate the causation of the symptoms either on a temporary or permanent basis, or to prevent or slow the appearance of symptoms of the named disorder or condition.
12) "Therapeutically effective amount" means a quantity of the compound which is effective in treating the named disorder, disease or condition.
13) "Pharmaceutically acceptable carrier" is a non-toxic solvent, dispersant, excipient, adjuvant or other material which is mixed with the active ingredient in order to permit the formation of a pharmaceutical composition, i.e., a dosage form capable of administration to the patient. One example of such a carrier is a pharmaceutically acceptable oil typically used for parenteral administration.
14) "Glucoregulatory abnormalities" means abnormalities in glucose regulation. Such abnormalities are associated with a variety of diseases or conditions including, excessive weight gain and obesity, hyperglycemia, glucose intolerance, type 2 diabetes mellitus, diabetic ketoacidosis and hypertriglyceridemia.
15) "Antipsychotic agents" refers to antipsychotic medications that have metabolic side effects. Such agents include, Clozapine, Olanzapine, Risperidone, Quetiapine, Haloperidol and Fluphenazine.
16) "Schizophrenia" is a disorder characterized by a mixture of signs and symptoms (both positive and negative). These signs and symptoms are associated with marked social or occupational dysfunction. They involve a range of cognitive and emotional dysfunctions that include perception, inferential thinking, language and communication, behavioral monitoring, affect, fluency and productivity of thought and speech, hedonic capacity, volition and drive, and attention.

Characteristic symptoms fall into two broad categories: positive and negative. Positive symptoms reflect an excess or distortion of normal functions. Negative symptoms reflect a diminution or loss of normal functions. Positive symptoms include the following: distortions in thought content (delusions), perception (hallucinations), language and thought process (disorganized speech), and self-monitoring of behavior (grossly disorganized or catatonic behavior). Negative symptoms include the following: restrictions in the range and intensity of emotional expression (affective flattening), in the fluency and productivity of thought and speech (alogia), and in the initiation of goal-directed behavior (avolition).

Particularly preferred are compounds wherein R is hydrogen, halogen, trifluoromethyl, or C₁-C₆alkyl; R₁ is hydrogen or C₁-C₆alkyl; R₂ is hydrogen or C₁-C₆alkyl; R₃ is hydrogen, C₁-C₆alkyl or halogen; and p is 0. Further preferred compounds are those wherein the amino group is attached to the 4-position of the pyridine group. Also preferred are compound of Formula I wherein R3 is amino and is attached to the 3-position of the pyridine group.

Even more particularly preferred are the compounds of formulas II [also known herein as HP184 or N-(3-fluoro-4-pyridinyl)- N-propyl-3-methyl-1H-indole-1-amine] and III (also known herein as "8183"). The compounds of formulas IV, V, VI and VII are also particularly preferred.

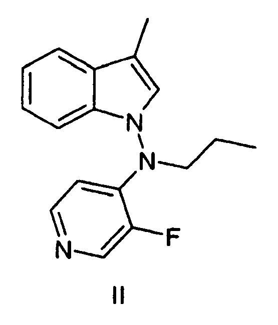

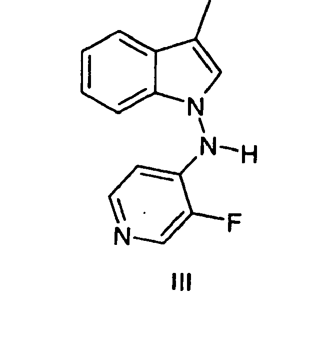

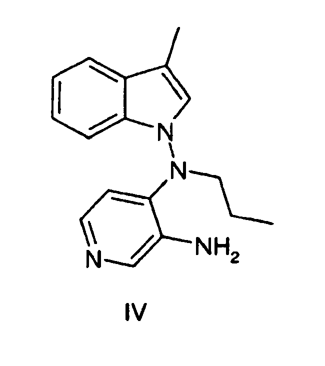

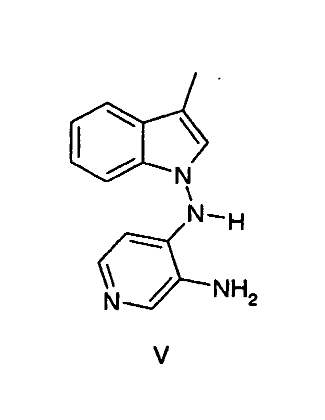

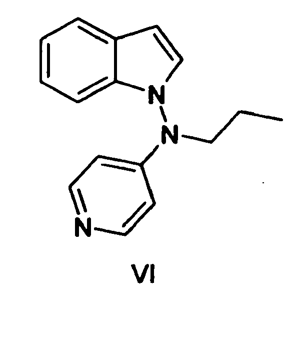

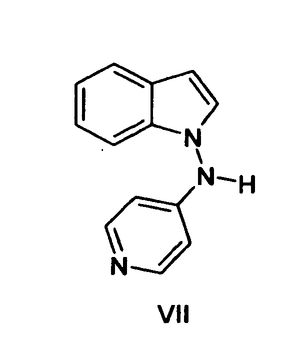

The compounds used in the methods claimed herein can be synthesized via procedures disclosed in United States Patent No. 4,970,218.

With the ever-increasing evidence of white matter impairment in schizophrenia over the last 6 years, the methods claimed herein should have efficacy in schizophrenia, based on the effects of the compounds of formula I on conduction velocity and white matter repair.

HP184, for example, increases conduction velocity and may increase myelination in schizophrenia. HP184 and other compound of formula I would therefore be expected to improve cognition, decrease negative symptoms and improve functional outcome in patients with residual symptoms after treatment with other antipsychotic medications. Without wishing to be bound by theory, it has been hypothesized that residual symptoms and cognitive impairment are due to decreased conduction velocity, preventing antipsychotic drugs from being fully effective. Other mechanisms of HP 184, such as enhanced release of cortical dopamine (prefrontal cortex), acetylcholine (temporal lobe) and noradrenaline are also expected to play a role in the hypothesized cognitive and clinical improvements. As schizophrenia is associated with altered but not chronically deteriorating or dying neurons, improvement of conduction should be possible with improvement in cognitive impairment and negative symptoms.

It has now been discovered that compounds of formula I block the Kv2.1 and Kv1.3 channels. Compounds with such activity should provide useful agents to treat glucoregulatory abnormalities such as hyperglycemia and type 2 diabetes mellitus. Such compounds should also be useful to treat the schizophrenic patient population that has been identified as having an increased risk of developing diabetes. The differences in glucose regulation in the schizophrenic patient population may be related to the disease itself or they may be medication-related.

Previously, HP184 and other compounds of Formula I have been shown to inhibit potassium currents in PC12 cells. This blockade is consistent with a voltage-dependent block, and has not been fully characterized. Recently, applicants tested HP184 to see if the compound could block Kv2.1 and Kv1.3 channels. As stated earlier herein, blockade of Kv2.1 channels has been linked to enhancement of glucose-stimulated insulin release. In the examples that follow, HP184 is shown to block voltage-activated Kv2.1 channels in both Syrian Hamster Insulinoma Cells (HIT-T15) and U-373MG cells expressing the human Kv2.1 channel. The magnitude of the blockade result was rather surprising since 4-amino pyridine (i.e. "4-AP"), another potassium channel blocker, showed a much lower affinity for the human Kv2.1 channel. Furthermore, as previously disclosed, HP184 affects neurotranmsitter release in a different way than 4-AP. From rat brain slices, HP184 enhances the release of norepinephrine, acetylcholine and serotonin in the absence of added calcium, whereas 4-AP's neurotransmitter release enhancing properties are dependent on added calcium (Smith et al, 1993; 1996).

It is also interesting to note that HP184 does not interact with muscarinic, α₂-adrenergic or serotonin_{1A} receptors, nor the noradrenergic or serotonergic uptake carriers (Smith et al, 1993; 1996). This lack of interaction at serotonin_{1A} receptor differentiates HP184 from several atypical antipsychotics which are believed to be responsible for the increased risk of hyperglycemia and diabetes in the schizophrenic patient population.

Recent experiments conducted by applicants suggest that HP184 also blocks Kv1.3 channels. As stated earlier, these channels have been shown to increase insulin sensitivity in mice (Xu et al, 2004). The examples that follow include data showing that HP184 blocks voltage-activated K+ currents present on human T-cells, previously activated eight times with CD3/CD28, which activates the T-cells immunologically (Panyi et al, 2003). Under these conditions, the predominant potassium channel is of the Kv1.3 type (Beeton et al, 2003).

Current type 2 diabetes treatments aimed at enhancing insulin secretion are limited to sulfonylurea drugs, which act in a glucose-independent manner. HP184 and other compounds of Formula I may be effective as monotherapy agents, or alternatively as adjunct therapy to other agents that can be used to treat glucoregulatory abnormalities, such as for example, type 2 diabetes agents.

In treating a patient afflicted with a condition or disorder described above, a compound of formula (I) can be administered in any form or mode which makes the compound bioavailable in therapeutically effective amounts, including orally, sublingually, buccally, subcutaneously, intramuscularly, intravenously, transdermally, intranasally, rectally, topically, and the like. One skilled in the art of preparing formulations can determine the proper form and mode of administration depending upon the particular characteristics of the compound selected for the condition or disease to be treated, the stage of the disease, the condition of the patient and other relevant circumstances. For example, see Remington's Pharmaceutical Sciences, 18th Edition, Mack Publishing Co. (1990).

The compounds of Formula I may be administered as monotherapy or adjunct therapy. The compounds of Formula I can be administered alone or in the form of a pharmaceutical composition in combination with pharmaceutically acceptable carriers, the proportion and nature of which are determined by the solubility and chemical properties of the compound selected, the chosen route of administration, standard pharmaceutical practice and other relevant criteria. Further active ingredients may be combined with the compounds of the formula I in particular for a synergistic improvement of the effect. Administration of the active ingredient combination may take place either by separate administration of the active ingredients to the patient or in the form of combination products in which a plurality of active ingredients are present in one pharmaceutical preparation.

Further active ingredients that may be combined with compounds of the formula I include antidiabetics agents. These agents include insulin and insulin derivatives such as, for example, Lantus^{®} (see www.lantus.com) or HMR 1964, fast-acting insulins (see US 6,221,633), GLP-1 derivatives such as, for example, those disclosed in WO 98/08871 of Novo Nordisk A/S, and orally effective hypoglycemic active ingredients.

The orally effective hypoglycemic active ingredients include, preferably, sulfonylureas, biguanidines, meglitinides, oxadiazolidinediones, thiazolidinediones, glucosidase inhibitors, glucagon antagonists, GLP-1 agonists, potassium channel openers such as, for example, those disclosed in WO 97/26265 and WO 99/03861 of Novo Nordisk A/S, insulin sensitizers, inhibitors of liver enzymes involved in the stimulation of gluconeogenesis and/or glycogenolysis, modulators of glucose uptake, compounds which alter lipid metabolism, such as antihyperlipidemic active ingredients and antilipidemic active ingredients, compounds which reduce food intake, PPAR and PXR agonists and active ingredients which act on the ATP-dependent potassium channel of the beta cells.

The compounds of the present invention may be administered orally, for example, in the form of tablets, troches, capsules, elixirs, suspensions, solutions, syrups, wafers, chewing gums and the like and may contain one or more of the following adjuvants: binders such as microcrystalline cellulose, gum tragacanth or gelatin; excipients such as starch or lactose, disintegrating agents such as alginic acid, Primogel, corn starch and the like; lubricants such as magnesium stearate or Sterotex; glidants such as colloidal silicon dioxide; and sweetening agents such as sucrose or saccharin may be added or a flavoring agent such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the present compounds, sucrose as a sweetening agent and certain preservatives, dyes and colorings and flavors.

The compounds of Formula (I) of this invention may also be administered topically, and when done so the carrier may suitably comprise a solution, ointment or gel base. The base, for example, may comprise one or more of petrolatum, lanolin, polyethylene glycols, bee wax, mineral oil, diluents such as water and alcohol, and emulsifiers and stabilizers.

The solutions or suspensions may also include one or more of the following adjuvants: sterile diluents such as water for injection, saline solution, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as ethylene diaminetetraacetic acid; buffers such as acetates, citrates or phosphates and agents for the adjustment of tonicity such as sodium chloride or dextrose. The parenteral preparation can be enclosed in ampules, disposable syringes or multiple dose vials.

The highly lipophilic esters, amides and carbamate derivatives of the present invention are capable of sustained release in mammals for a period of several days or from about one to four weeks when formulated and administered as depot preparations, as for example, when injected in a properly selected pharmaceutically acceptable oil. The preferred oils are of vegetable origin such as sesame oil, cottonseed oil, corn oil, coconut oil, soybean oil, olive oil and the like, or they are synthetic esters of fatty acids and polyfunctional alcohols such as glycerol or propyleneglycol.

The depot compositions of the present invention are prepared by dissolving a highly lipophilic ester, amide or carbamate derivative of a compound of formula I in a pharmaceutically acceptable oil under sterile conditions. The oil is selected so as to obtain a release of the active ingredient over a desired period of time. The appropriate oil may easily be determined by consulting the prior art, or without undue experimentation by one skilled in the art.

The dosage range at which the compounds of Formula I exhibit their ability to act therapeutically can vary depending upon the particular disease or condition being treated and its severity, the patient, the formulation, other underlying disease states that the patient is suffering from, and other medications that may be concurrently administered to the patient. Generally, the compounds of Formula I will exhibit their therapeutic activities at dosages of between about 0.001 mg/kg of patient body weight/day to about 100 mg/kg of patient body weight/day.

The following examples are for illustrative purposes.

### EXAMPLES

### EXAMPLE I

### EFFECT OF HP184 ON VOLTAGE-GATED POTASSIUM CHANNELS IN HAMSTER INSULINOMA CELLS

The purpose of this in vitro study was to evaluate the effects of HP184 on voltage-gated potassium channels in hamster HIT-T15 insulinoma cells using the whole-cell patch-clamping technique.

HIT-T15 cells (from Syrian Hamster pancreas) expressing voltage-gated potassium channels were grown in RPMI media supplemented with 10% fetal bovine serum and 1X penicillin/streptomycin in an atmosphere of 95% air/5% CO₂. Cells used for patch-clamping were seeded on glass or plastic coverslips 12-36 hours before use. Currents were recorded at room temperature using the whole-cell configuration of the patch clamp technique with an Axopatch 200B amplifier (Axon Instruments, Foster City, CA). Briefly, electrodes (3-6 MΩ resistance) were fashioned from TW150F glass capillary tubes (World Precision Instruments, Sarasota, FL) and filled with pipette solution (in mM: potassium aspartate 120; KCl 20; Na₂ATP 4; HEPES 5; MgCl₂ 1; pH 7.2 adjusted with KOH). Currents were initiated by a 300-ms positive voltage pulse (20 mV) followed by a 50-ms negative pulse (-100 mV) and were recorded for off-line analyses. Once current from a cell perfused with control external solution (in mM: NaCl 130; KCl 5; sodium acetate 2.8; MgCl₂ 1; HEPES, 10; glucose 10; CaCl₂ 1 at pH7.4 adjusted with NaOH) was stabilized, the cell was perfused with external solution containing 1 µM HP184 (batch: R.C.4.53.3; molecular weight: 320.5; diluted from a 50 mM stock solution in DMSO prepared daily; the DMSO final concentration in all drug solutions was not more than 0.06%). Currents were continuously recorded until they reached steady-state condition. The same procedure was performed with increasing concentrations of HP184 (3, 10 and 30 µM, sequentially). For each concentration from each cell, the steady-state current at the end of the 20-mV activation pulse was measured in pico Ampere (pA).

The amplitude in pA for each concentration was compared with that for the control solution from the same cell and expressed as percent control (% control).

The effect of HP184 on the voltage-gated potassium currents in hamster HIT-T15 insulinoma cells is summarized in Table 1. The compound blocked the currents dose-dependently with an IC₅₀ value of 3.9 µM (Figure 1).

**Table 1. Effects of HP184 on the voltage-gated potassium currents in HIT-T15 cells**

| **concentration (µM)** | **Voltage-Gated Potassium Current (%Control)** | | | | | | |
|---|---|---|---|---|---|---|---|
| | Cell #1 | Cell #2 | Cell #3 | Cell #4 | Cell #5 | Average | SEM |
| **0** | 100 | 100 | 100 | 100 | 100 | 100 | 0.0 |
| **1** | 95.1 | 84.7 | 80.9 | 85.4 | 82.6 | 85.7 | 2.5 |
| **3** | 86.4 | 61.5 | 46.4 | 58.8 | 59.3 | 62.5 | 6.5 |
| **10** | 34.0 | 9.1 | 12.6 | 17.8 | 12.6 | 17.2 | 4.4 |
| **30** | 2.6 | | 0.8 | 0.9 | 1.2 | 1.4 | 0.4 |

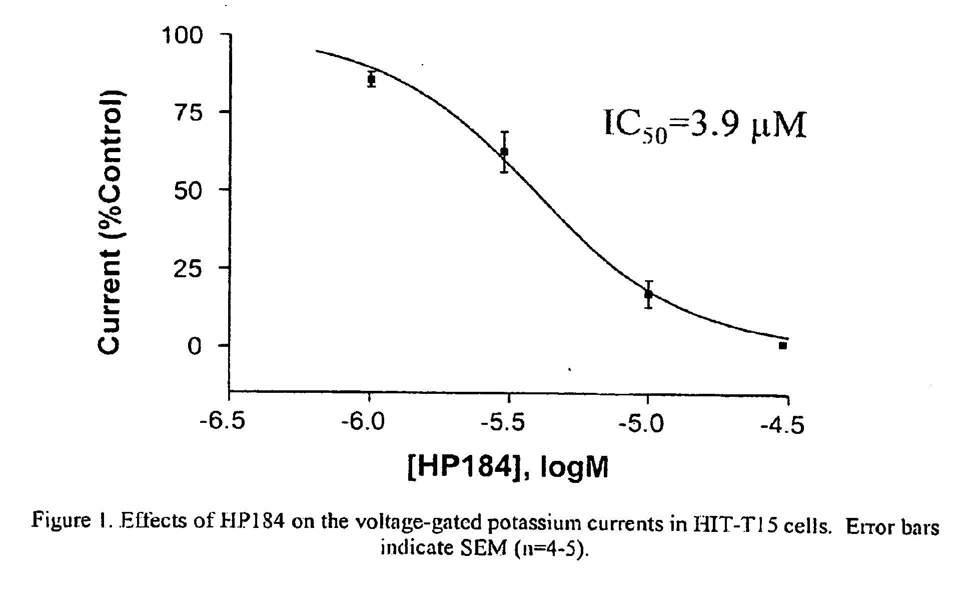

### EXAMPLE II

### THE EFFECT OF HP184 ON CLONED HUMAN KV2.1 POTASSIUM CHANNEL

The purpose of this in vitro study was to evaluate the effects of HP184 on cloned human Kv2.1 potassium channels expressed in U-373MG cells using the whole-cell patch-clamping technique.

U-373MG cells expressing the human Kv2.1 channels were grown in DMEM media supplemented with 10% fetal bovine serum, 1X penicillin/streptomycin and 500 mg/mL G418 (Invitrogen, Carlsbad, CA) in an atmosphere of 95% air/5% CO₂. Cells used for patch-clamping were seeded on glass or plastic coverslips 12-36 hours before use. Kv2.1 Currents were recorded at room temperature using the whole-cell configuration of the patch clamp technique with an Axopatch 200B amplifier (Axon Instruments, Foster City, CA). Briefly, electrodes (2-4 MΩ resistance) were fashioned from TW150F glass capillary tubes (World Precision Instruments, Sarasota, FL) and filled with pipette solution (in mM: potassium aspartate 120; KCl 20; Na₂ATP 4; HEPES 5; MgCl₂ 1; pH 7.2 adjusted with KOH). For dose-dependency, currents were initiated by a 300-ms positive voltage pulse (20 mV) followed by a 50-ms negative pulse (-100 mV) and were recorded for off-line analyses. Once current from a cell perfused with control external solution (in mM: NaCl 130; KCl 5; sodium acetate 2.8; MgCl₂ 1; HEPES, 10; glucose 10; CaCl₂ 1 at pH7.4 adjusted with NaOH) was stabilized, the cell was perfused with external solution containing 1 µM HP184 (batch: R.C.4.53.3; molecular weight: 320.5; diluted from a 50 mM stock solution in DMSO; the DMSO final concentration in all drug solutions was not more than 0.06%). Currents were continuously recorded until they reached steady-state condition. The same procedure was performed with increasing concentrations of HP184 (3, 10 and 30 µM, sequentially). For each concentration from each cell, the steady-state current at the end of the 20-mV activation pulse was measured in pico Ampere (pA). The amplitude in pA for each concentration was compared with that for the control solution from the same cell and expressed as percent control (% control). For comparison, 4-aminopyridine (4-AP) was tested in a similar manner at concentrations ranging from 100 to 10, 000 µM. The effect of HP184 and 4-AP on the human Kv2.1 currents expressed in U-373MG cells is summarized in Table 1 and 2, respectively. HP184 blocked the Kv2.1 current dose-dependently with an IC₅₀ value of 5.6 µM, while 4-AP blocked the current with an IC50 >10,000 µM (Figure 1).

**Table 1. Effects of HP184 on Cloned Human Kv2.1 Currents**

| **Concentration (µM)** | **Kv2.1 Current (%Control)** | | | | | | | | | | | | | | |
|---|---|---|---|---|---|---|---|---|---|---|---|---|---|---|---|
| | Cell #1 | Cell #2 | Cell #3 | Cell #4 | Cell #5 | Cell #6 | Cell #7 | Cell #8 | Cell #9 | Cell #10 | Cell #11 | Cell #12 | Cell #13 | **Average** | SEM |
| **0** | 100 | 100 | 100 | 100 | 100 | | | | 100 | | 100 | | 100 | **100** | 0.0 |
| **1** | 96.0 | 91.6 | 95.0 | 90.5 | 94.S | 83.5 | 91.8 | 95.5 | 90.5 | 91.0 | 89.2 | | | **91.7** | 1.1 |
| **3** | 93.0 | 62.9 | 68.5 | 70.8 | 87.5 | 65.1 | 68.5 | 79.9 | 65.7 | 63.1 | 69.9 | | | **72.3** | 3.1 |
| **10** | 58.2 | | 24.4 | 17.1 | 38.5 | 43.8 | 13.1 | 19.1 | 12.5 | 19.0 | 37.7 | | | **28.3** | 4.9 |
| **30** | 22.1 | | | 1.4 | 12.6 | 13.7 | 4.6 | 8.7 | 4.6 | | 13.7 | 16.2 | 6.7 | **10.4** | 2.0 |

**Table 2. Effects of 4-AP on Cloned Human Kv2.1 Currents**

| **Concentration (µM)** | **Kv2.1 Current (%Control)** | | | | | | | | | | | |
|---|---|---|---|---|---|---|---|---|---|---|---|---|
| | Cell #1 | Cell #2 | Cell #3 | Cell #4 | Cell #5 | Cell #6 | Cell #7 | Cell #8 | Cell #9 | Cell #10 | **Average** | SEM |
| **0** | 100 | 100 | 100 | 100 | 100 | | | | 100 | | **100** | 0.0 |
| **100** | 90.2 | 94.3 | 95.9 | 92.0 | 92.9 | 83.8 | | | | | **91.5** | 1.7 |
| **300** | | 88.9 | 87.8 | 85.0 | 82.8 | 73.1 | | | | | **83.5** | 2.8 |
| **1000** | 79.0 | 81.4 | 78.9 | 76.8 | | 60.4 | 70.8 | 71.8 | 85.0 | 81.5 | **76.2** | 2.5 |
| **3000** | 74.0 | 74.4 | 60.0 | 69.5 | | 54.7 | 59.2 | 64.0 | 69.1 | 56.8 | **65.6** | 2.4 |
| **10000** | | 66.7 | 59.0 | | | | 46.8 | | | | **57.8** | 5.9 |

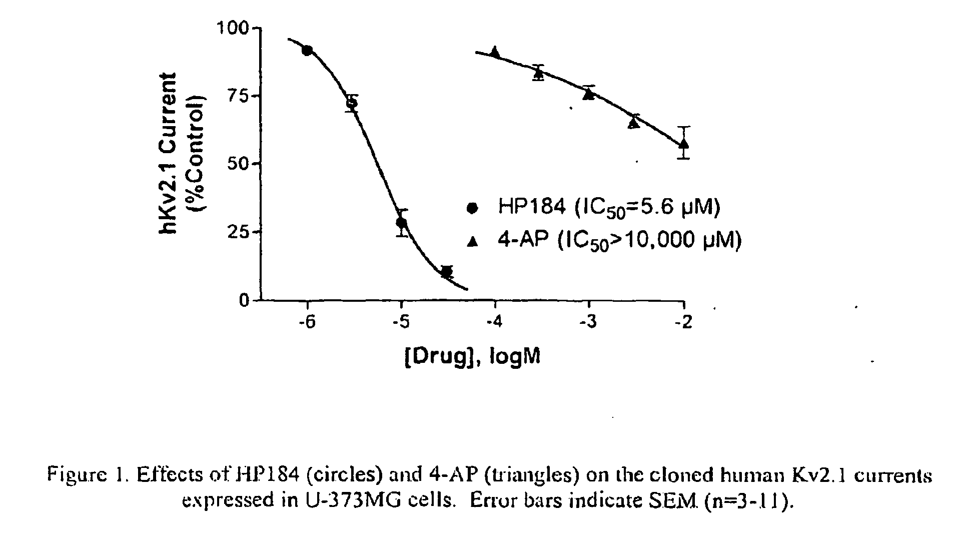

### EXAMPLE III

The purpose of this in vitro study was to evaluate the effect of HP184 on voltage-gated potassium channels in activated T cells using the whole-cell patch-clamping technique.

Human T cells activated with CD3/CD28 (8X) by Melinda Cilio (IP Cellular Immunology) were seeded on plastic coverslips 12-36 hours before use. Voltage-gated potassium channel currents were recorded at room temperature using the whole-cell configuration of the patch clamp technique with an Axopatch 200B amplifier (Axon Instruments, Foster City, CA). Briefly, electrodes (3-6 MΩ resistance) were fashioned from TW150F glass capillary tubes (World Precision Instruments, Sarasota, FL) and filled with pipette solution (in mM: potassium aspartate 120; KCl 20; Na₂ATP 4; HEPES 5; MgCl₂ 1; pH 7.2 adjusted with KOH). The potassium currents were initiated by a 100-ms 20-mV voltage pulse from a holding potential of-80 mV and were recorded for off-line analyses. Once the current from the cell perfused with control external solution (in mM: NaCl 130; KCl 5; sodium acetate 2.8; MgCl₂ 1; HEPES, 10; glucose 10; CaCl₂ 1 at pH7.4 adjusted with NaOH) was stabilized, the cell was perfused with external solution containing 1 µM HP184. Currents were continuously recorded until they reached steady-state condition. The same procedure was performed with 10 µM HP184. The data is shown in the figure below.

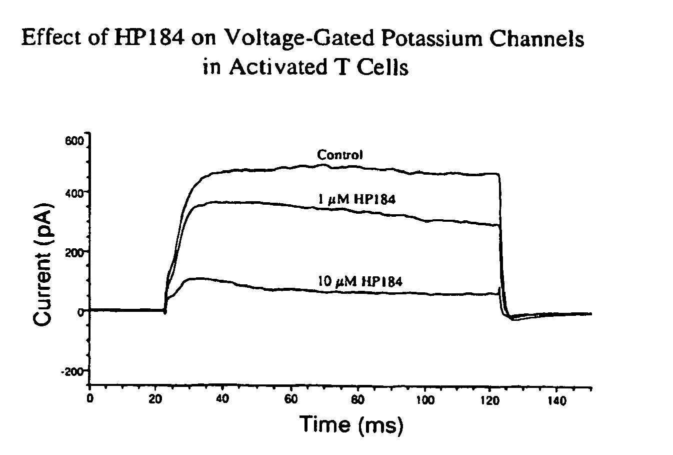

### REFERENCES

1. Agartz I, Andersson JL, Skare S. Abnormal brain white matter in schizophrenia: a diffusion tensor imaging study. NeuroReport. 12:2251-2254, 2001
2. Altamura C, Guercetti G, Percudani M. Dexamethasone suppression st in positive and negative schizophrenia. Psychiatry Res.30:69-75, 1989
3. Andreasen NC, Nopoulos P, O'Leary DS, Miller DD, Wassink T, Flaum M. Defining the phenotype of schizophrenia: cognitive dysmetria and its neural mechanisms. Biological Psychiatry, 46, 908-920, 1999.
4. Bartzokis G. Schizophrenia: breakdown in the well-regulated lifelong process of brain development and maturation. Neuropsychopharmacology 27:672-683,2002
5. Bartzokis et al. Biol Psychiatry 2003
6. Bellack AS, Schooler NR, Marder SR, et al. Do Clozapine and risperidone affect social competence and problem solving? Am J Psychiatry. 161:364-367, 2004
7. Benes
8. Beng-Choon et al 2003
9. Bilder RM,Goldman RS, Robinson D, Reiter G, Bell L, Bates JA, et al., (2000). Neuropsychology of first-episode schizophrenia: initial characterization and clinical correlates. American Journal of Psychiatry, 157, 549-559.
10. Bleuler E. Dementia Praecox or the Group of Schizophrenias. Zinkin J (translat). New York, NY: International Universities Press; 1950
11. Buchsbaum MS, Ingvar DH, Kessler R, Waters RN, Cappelletti J, van Kammen DP, King AC, Johnson JL, Manning RG, Flynn RW, Mann LS, Bunney WE Jr, Sokoloff L. Cerebral glucography with positron tomography: Use in normal subjects and in patients with schizophrenia. Arch Gen Psychiatry, 39:251-260, 1982.
12. Buchsbaum MS, Tang CY, Peled S, Gudbjartsson H, Lu D, Hazlett E A, Downhill J, Haznedar M, Fallon JH, Atlas SW. (1998). MRI white matter diffusion anisotropy and PET metabolic rate in schizophrenia. Neuroreport, 9, 425-430
13. Carter CS, McDonald AW, Ross LL, Stenger VA. (2001). Anterior cingulate cortex activity and impaired self-monitoring of performance in patients with schizophrenia: An event related fMRI study. American Journal of Psychiatry, 158, 1423-1428
14. Cleghorn JM, Franco S, Szechtman B, et al. Toward a brain map of auditory hallucinations. Am J Psychiatry. 149:1062-1069, 1992
15. Cotter D, Mackay D, Beasley C, Kervin R, Everall I. Reduced glial cell density and neuronal volume in major depressive disorder and schizophrenia in the anterior cingulate cortex. See ref 15 2000
16. Davis KL, Stewart DG, Friedman JI, et al. White matter changes in schizophrenia. Arch Gen Psychiatry. 60: 443-456, 2003
17. Deicken et al 1994
18. Egan et al, 1994
19. Everett et al 1989;
20. Flyn SW, Lang DJ, Mackay AL, et al. Abnormalities of myelination in schizophrenia detected in vivo with MRI and post-mortem with analysis of oligodendrocyte proteins. Molecular Psychiatry. 8:811-820, 2003
21. Foong J, Maier M, Barker G J, Brocklehurst, Miller DH, Ron MA. In vivo investigation of white matter pathology in schizophrenia with magnetization transfer imaging. Journal of Neurology, Neurosurgery, and Psychiatry, 68, 70-74, 2000.
22. Foong J, Symms MR, Barker GJ, et al. Investigating regional white matter in schizophrenia using diffusion tensor imaging. NeuroReport 13:333-336, 2002
23. Friston KJ. The dysconnection hypothesis. Schizophrenia Res. 30:115-125, 1998
24. Gilbertson MW, Yao JK, van Kammen DP. Memory and plasma HVA changes in schizophrenia: Are they episode markers? Biol Psychiatry, 35:203-206, 1994.
25. Goetz K, van Kammen DP. Computerized axial tomography scans and subtypes of schizophrenia: A review of the literature. J Nerv Ment Dis, 174:31-41, 1985
26. Goldman-Rakic PS, Selemon LD, Schwartz ML (1984): Dual pathways connecting the dorsolateral prefrontal cortex with the hippocampal formation and parahippocampal cortex in the rhesus monkey. Neuroscience 12:719-43.
27. Goldstein G, Allen DN, van Kammen DP. Brief report: Individual differences in cognitive decline in schizophrenia. Am J Psychiatry, 155:1117-1118, 1998
28. Green, MF. What are the functional consequences of the cognitive deficit in schizophrenia? Am J Psychiatry.153: 321-330, 1996
29. Green, MF. Interventions for cognitive deficits: editor's introduction. Schizophrenia Bull. 25:197-200, 1999
30. Higashima et al, 1998
31. Hoff PR, Haroutunian V, Friedreich Jr. VL, Byne W, Buitron C, Perl DP, Davis KL. Loss and altered spatial distribution of oligodendrocytes in the superior frontal gyrus in schizophrenia. Biol Psychiatry. 53:1075-1085, 2003
32. Hoptman MJ, Volavka J, Johnson G, Weiss E, Bilder RM, Lim KO. Frontal White matter microstructure, aggression, and impulsivity in men with schizophrenia: a preliminary study. Biol Psychiatry. 52:9-14, 2002
33. Iwanami A, Okajima Y, Kuwakado D, et al. Event-related potentials and thought disorder in schizophrenia. Schizophrenia Res. 42:187-191, 2000
34. Jakob and Beckman,
35. Juckel et al 1996
36. Kay SR, Opler LA, Fiszbein A. Positive and negative symptom scale (PANSS). New York: Bronx Psychiatric Center. 1986
37. Keshavan et al., 1998
38. Kraepelin E. Dementia praecox and Paraphrenia. Barclay, trans. Edinburgh, Scotland: E&S Livingstone; 1999
39. Kubicki M, Westin C, Frumin M, Nestor P, Salisbury D, Kikinis R, Jolesz F, McCarley R, Shenton M. (2002). Uncinate fasciculus findings in schizophrenia: a magnetic resonance diffusion tensor imaging study. Am J Psychiatry, 159, 813-820.
40. Kubicki, M., Westin, C.F., Nestor, P.G., Wible, C.G., Frumin, M., Maier, S.E., Kikinis, R., Jolesz, F.A., McCarley, R.W., & Shenton, M.E. Cingulate fasciculus integrity disruption in schizophrenia: A magnetic resonance diffusion tensor imaging study. Biological Psychiatry 54:1171-1180, 2003
41. Lewis D
42. Liddle PF. Functional imaging: schizophrenia. Br. Med Bull. 52:486-494, 1996
43. Lim KO, Hedehus M, Moseley M, de Crespigny A, Sullivan EV, Pfefferbaum A. Compromised white matter tract integrity in schizophrenia inferred from diffusion tensor imaging. Arch Gen Psychiatry. 56:367-374, 1999
44. Linszen DH, Nieman DH. Cannabis use adversely affects p300 event-related potential latency in recent-onset schizophrenia. Schizophrenia Res. 67:218-219,2004.
45. McCarley et al., 1997
46. McGurk SR and Meltzer HY The role of cognition in vocational functioning in schizophrenia. Schizophrenia Res. 2000; 435:175-184, 1999, 25: 233-255
47. McGuire, P. K., & Frith, C. D. (1996). Disordered functional connectivity in schizophrenia. Psychological Medicine 26, 663-667
48. Meltzer HY, and McGurk SR. The effects of clozapine, risperidone and olanzapine on cognitive function in schizophrenia. Schizophrenia Bulletin. 1999, 25: 233-255
49. Miyakawa T, SumiyoshS, Deshimaru M et al. Electron microscopic study on schizophrenia: mechanisms of pathological changes. Acta Neuropathol. 20:67-77, 1972
50. Murray R, Lewis SR. Is schizophrenia a developmental disorder? Br Med J. 295:681-682, 1987
51. Nieman et al 2002
52. Nestor PG, Kubicki M, GurreraRJ, Niznikiewicz M, Frumin M, McCarley RW, Shenton ME. Neuropsychology of diffusion tensor imaging brain connectivity in schizophrenia. Neuropsychology, In Press
53. Nestor PG, Akdag SJ, O'Donnell BF, et al. (1998b). Word recall in schizophrenia: a connectionist model. Am J Psychiatry, 155, 1685-1690.
54. Newcomer JW, Faustman O, Whiteford HA, et al. Symptomatology and cognitive impairment associate independently with post-dexamethasone cortisol concentrations in unmedicated schizophrenic patients. Biol Psychiatry 29:855-846, 1991
55. Orlovskaya DD, Vostrikov VM, Rachmanova VI, Uranova NA. Decreased numerical density of oligodendroglial cells in post-mortem prefrontal cortex in schizophrenia, bipolar affective disorder, and major depression. Schizophrenia Res. 41:105-106, 2000
56. Palmer et al. Is it possible to be schizophrenic yet neuropsychologically normal? Neuropsychology. 11: 437-446, 1999
57. Barta PE, Pearlson GD, Powers RE et al. Auditory hallucinations and smaller superior temporal gyral volume in schizophrenia. Am J Psychiatry. 14:1457-1462, 1990.
58. Rajkowska G, Selemon LD, Goldman-Rakic PS. Neuronal and glial somal size in the prefrontal cortex: a post-mortem study of schizophrenia and Huntington's disease. Arch Gen Psychiatry. 55:215-224, 1998.
59. Saykin AJ, Shtasel DL, Gur GE, et al. Neuropsychological deficits in neuroleptic naive patients with first-episode schizophrenia. Arch Gen Psychiatry, 51, 124-131, 1994.
60. Seaton BE, Allen DN, Goldstein G, Kelley ME, van Kammen DP. Cognitive heterogeneity in schizophrenia is not accounted for by symptom profile. J Nerv Ment Dis. 187:414-419, 1999
61. Selemon LD, Goldman-Rakic PS. The reduced neuropil hypothesis: a circuit based model of schizophrenia. Biol Psychiatry. 1999, 45:17-25
62. Shihabuddin et al 2000
63. Spaletta G, Tomaiuolo F, Marino V, Bonaviri G et al. Chronic schizophrenia as a brain misconnection syndrome: a white matter voxel-based morphometric study. Schizophrenia Res. 64:12-23, 2003
64. Strik et al 1993
65. Suzuki M, Nohara S, Hagino H, et al. Regional changes in gray and white matter in patients with schizophrenia demonstrated with voxel based analysis of MRI. Schizophrenia Res. 55: 41-54, 2002
66. Tkachev D, Mimmack ML, Ryan MM, et al. Oligodendrocyte dysfunction in schizophrenia and bipolar disorder. The Lancet. 362:798-805
67. Torrey EF, Webster W, Knable M, Johnston N, Yolken RH. The Stanley Foundation Brain Collection and Neuropathology Consortium. Schizophrenia Res. 44:151-155, 2000
68. Uranova NA, Vostrikov, VM, Orlovskaya DD, et al. Decreased density of oligodendroglial satellites of pyramidal neurons in layer III in the prefrontal cortex in schizophrenia and mood disorders. Schizophrenia Res. 53: 107, 2002
69. Uranova NA, Orlovskaya DD, Vikhreva O, et al. Electron microscopy of oligodendroglia in severe mental Illness. Brain Res Bull. 55:597-610, 2001,
70. Verdoux et al 2002Walder DJ, Walker EF, Lewine RJ. Cognitive functioning, cortisol release, and symptom severity in patients with schizophrenia. Biol Psychiatry. 48:1121-1132, 2000
73. Walker EF, Diforio D. Schizophrenia: a neural diathesis-stress model. Psychol Rev. 104:667-685, 1997
74. Weinberger DR. Arch Gen Psychiatry. 1986
75. Weinberger, DR, Berman KF, Illowsky BP. (1988). Physiological dysfunction of dorsolateral prefrontal cortex in schizophrenia, III: a new cohort and evidence for a monoaminergic mechanism. Archives of General Psychiatry. 45, 609-615.
76. Weinberger D, Berman KF, Rec R F. (1986). Physiological dysfunction of dorsolateral prefrontal cortex in schizophrenia, I: regional cerebral blood flow evidence. Archives of General Psychiatry, 43, 114-124.
77. Weinberger DR, Berman KF, Suddath R, Torrey EF. (1992). Evidence of dysfunction of a prefrontal -limbic network in schizophrenia: a magnetic resonance imaging and regional cerebral blood flow of discordant monozygotic twins. American Journal of Psychiatry, 149, 890-897
78. Wernicke C. (1906) Grundrisse der Psychiatrie. Leipzig, Theirne
79. Woodruff, P. W., Wright IC, Shuriquie N, Rushe T, Howard RJ, Graves M, Bullmore, ET. Structural brain abnormalities in male schizophrenics reflect fronto-temporal dissociation. Psychol Med. 27:1257-66;1997
80. Walker E, et al.
81. Wolkin A, et al, 1998
82. Wolkin A, et al, 2003

Smith, C.P., A.T. Woods, Corbett, R., S.M. Chesson, G.M. Bores, W.W. Petko, J.E.Roehr and S. Kongsamut. Serotonergic activity of HP 184: Does spontaneous release have a role? Neurochemical Research 21:573-583, 1996.
Smith, C.P., L.R. Brougham, F.P. Huger, L. Davis, J.T. Klein and R.C. Effland. HP 184 [N-(n-propyl)-N-(3-fluroro-4-pyridinyl)-1H-3-methylindol-1-amine hydrochloride]: In vitro spontaneous release of acetylcholine (ACh) and norepinephrine (NE). Drug Dev. Res. 30:203-212, 1993.
Roe MW, Worley JFIII, Mittal AA, Kuznetsov A, DasGupta S, Mertz RJ, Witherspoon SMIII, Blair N, Lancaster ME, McIntyre MS, Shehee WR, Dukes ID and Philipson LH: Expression and Function of Pancreatic β-Cell Delayed Rectifier K+ Channels. Journal of Biological Chemistry 271:32241-332246, 1996.
MacDonald PE, Ha XF, Wang J, Smulker SR, Sun AM, Gaisano HY, Salapatek AMF, Backx PH and Wheeler MB: Members of the Kv1 and Kv2 Voltage-Dependent K+ Channel Families Regulate Insulin Secretion. Molecular Endocrinology 15:1423-1435, 2001.
MacDonald PE, Sewing S, Wang J, Joseph JW, Smukler SR, Sakellaropoulos G, Wang J, Saleh MC, Chan CB, Tsushima RG, Salapatek AMF and Wheeler MB: Inhibition of Kv2.1 Voltage-dependent K+ Channels in Pancreatic β-Cells Enhances Glucoes-dependent Insulin Secretion. Journal of Biological Chemistry 277:44938-44945, 2002.
Xu J, Wang P, Li Y, Li G, Kaczmarek LK, Wu Y, Koni PA, Flavell RA and Desir GV: The voltage-gated potassium channel Kv1.3 regulates peripheral insulin sensitivity. Proc Natl Acad Sci USA 101:3112-3117,2004.
Beeton C, Wulff H, Singh S, Botsko S, Crossley G, Gutman GA, Cahalan MD, Pennington M and Chandy KG: A novel fluorescent toxin to detect and investigate Kv1.3 channel regulation in chronically actoivated T lymphocytes. Journal of Biological Chemistry 278:9928-37, 2003.
Xu J, Koni PA, Wang P, Li G, Kaczmarek L, Wu Y, Li Y, Flavell RA, and Desir GV: The voltage-gated potassium channel Kv1.3 regulates energy homeostasis and body weight. Human Molecular Genetics 12:551-559, 2003.
Panyi G, Bagdany M, Bodnar A, Vamosi G, Szentesi G, Jenei A, Matyus L, Varga S, Waldmann TA, Gaspar R and Damjanovich S:Colocalization and nonrandom distribution of Kv1.3 potassium channels and CD3 molecules in the plasma membrane of Human T lymphocytes. Proc. Natl. Acad. Sci. USA 100:2592-2597, 2003.

## Claims

1. A compound of formula I 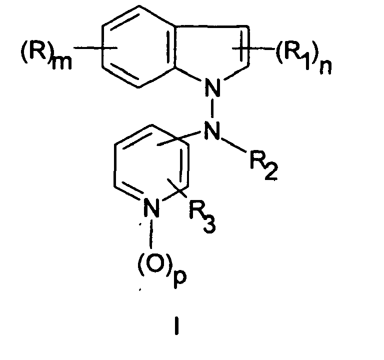 wherein
m is 0, 1 or 2;
n is 0, 1 or 2;
p is 0 or 1;
each R is independently hydrogen, halogen, trifluoromethyl, C₁-C₆alkyl, C₁-C₆alkoxy, benzyloxy, hydroxy, nitro or amino;
each R₁ is independently hydrogen, C₁-C₆alkyl, C₂-C₆alkenyl, C₁-C₆alkanoyl, halogen, cyano, -C(O)C₁-C₆alkyl, -C₁-C₆alkyleneCN, -C₁-C₆alkyleneNR'R" wherein R' and R" are each independently hydrogen or C₁-C₆alkyl, -C₁-C₆alkyleneOC(O)C₁-C₆alkyl, or -CH(OH)R₄ wherein R₄ is hydrogen or C₁-C₆alkyl;
R₂ is hydrogen, C₁-C₆alkyl optionally substituted with halogen, hydroxy or benzyloxy, C₂-C₆alkenyl, C₂-C₆alkynyl, -CO₂C₁-C₆alkyl, or -R₅-NR' R" wherein R₅ is C₁-C₆alkylene, C₂-C₆alkenylene or C₂-C₆alkynylene and R' and R" are each independently hydrogen, C₁-C₆alkyl or alternatively the group -NR'R" as a whole is 1-pyrrolidinyl; and R₃ is hydrogen, nitro, amino, halogen, C₁-C₆alkoxy, hydroxy or C₁-C₆alkyl
or a pharmaceutically acceptable salt thereof,
for use in the treatment of schizophrenia in a human.

2. A compound of formula I as defined in claim 1 or a pharmaceutically acceptable salt thereof for use in treating schizophrenia in a human while avoiding the concomitant liability of glucoregulatory abnormalities associated with the administration of antipsychotic agents.

3. A compound of formula I as defined in claim 1 or a pharmaceutically acceptable salt thereof for use in treating the cognitive dysfunction associated with schizophrenia in a human while avoiding the concomitant liability of glucoregulatory abnormalities associated with the administration of antipsychotic agents.

4. A compound of formula I as defined in claim 1 or a pharmaceutically acceptable salt thereof for use in treating the cognitive dysfunction associated with schizophrenia in a human while avoiding the concomitant liability of schizophrenia-related abnormalities in glucose regulation.

5. A compound of formula I as defined in claim 1 or a pharmaceutically acceptable salt thereof for use in treating the cognitive dysfunction and glucoregulatory abnormalities associated with schizophrenia in a human.

6. A compound of formula I as defined in claim 1 or a pharmaceutically acceptable salt thereof for use in treating glucoregulatory abnormalities in a patient.

7. A compound of formula I as defined in claim 1 or a pharmaceutically acceptable salt thereof for use in treating the negative symptoms associated with schizophrenia in a human while avoiding the concomitant liability of glucoregulatory abnormalities associated with the administration of antipsychotic agents

8. A compound of formula I as defined in claim 1 or a pharmaceutically acceptable salt thereof for use in enhancing glucose-stimulated insulin release in a patient.

9. The compound for use as claimed in claims 1-8 wherein R is hydrogen, halogen, trifluoromethyl, or C₁-C₆alkyl; R₁ is hydrogen or C₁-C₆alkyl; R₂ is hydrogen or C₁-C₆alkyl; R₃ is hydrogen, C₁-C₆alkyl or halogen; and p is 0.

10. The compound for use as claimed in claims 1-8 wherein the compound has the following formula

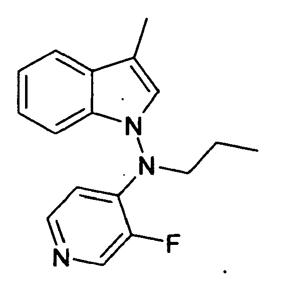

11. The compound for use as claimed in claims 1-8 wherein the compound has the following formula:

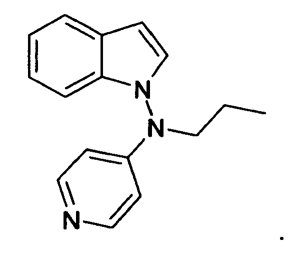

12. The compound for use as claimed in claim 6 wherein the glucoregulatory abnormality is hyperglycemia.

13. The compound for use as claimed in claim 6 wherein the glucoregulatory abnormality is type 2 diabetes mellitus.

14. A product comprising (i) a therapeutically effective amount of a compound of formula I as defined in Claim 1 or a pharmaceutically acceptable salt thereof, in combination with (ii) a therapeutically effective amount of an antipsychotic agent for simultaneous, separate, or sequential use in the treatment of schizophrenia in a human while avoiding the concomitant liability of glucoregulatory abnormalities associated with the administration of antipsychotic agents.

15. The product for use as claimed in claim 14 wherein R is hydrogen, halogen, trifluoromethyl, or C₁-C₆alkyl; R₁ is hydrogen or C₁-C₆alkyl; R₂ is hydrogen or C₁-C₆alkyl; R₃ is hydrogen, C₁-C₆alkyl or halogen; and p is 0.

16. The product for use as claimed in claim 14 wherein the compound has the following formula

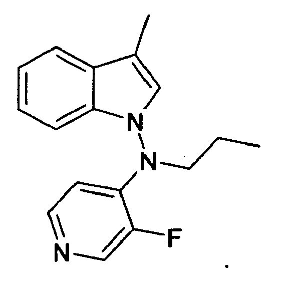

17. The product for use as claimed in claim 14 wherein the compound has the following formula:

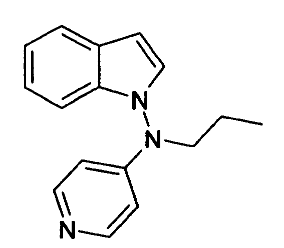

18. A product comprising (i) a therapeutically effective amount of a compound of formula I as defined in Claim 1 or a pharmaceutically acceptable salt thereof, in combination with (ii) a therapeutically effective amount of at least one other active ingredient selected from the group consisting of:
antidiabetics, hypoglycemic active ingredients, HMGCoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients which act on the ATP-dependent potassium channel of the beta cells, CART agonists, NPY agonists, MC4 agonists, orexin agonists, H3 agonists, TNF agonists, CRF agonists, CRF BP antagonists, urocortin agonists, β3 agonists, MSH (melanocyte-stimulating hormone) agonists, CCK agonists, serotonin reuptake inhibitors, mixed sertoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines,
for simultaneous, separate or sequential use in the treatment of glucoregulatory abnormalities in a patient.

## Patentansprüche

1. Verbindung der Formel I 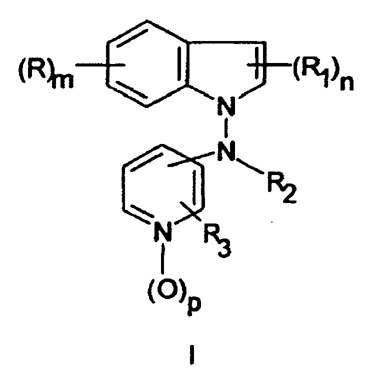 worin
m für 0, 1 oder 2 steht;
n für 0, 1 oder 2 steht;
p für 0 oder 1 steht;
R jeweils unabhängig voneinander für Wasserstoff, Halogen, Trifluormethyl, C₁-C₆-Alkyl, C₁-C₆-Alkoxy, Benzyloxy, Hydroxy, Nitro oder Amino steht;
R₁ jeweils unabhängig voneinander für Wasserstoff, C₁-C₆-Alkyl, C₂-C₆-Alkenyl, C₁-C₆-Alkanoyl, Halogen, Cyano, -C(O)-C₁-C₆-Alkyl, -C₁-C₆-Alkylen-CN, -C₁-C₆-Alkylen-NR'R'', worin R' und R'' jeweils unabhängig voneinander Wasserstoff oder C₁-C₆-Alkyl bedeuten, -C₁-C₆-Alkylen-OC(O)-C₁-C₆-alkyl oder -CH(OH)R₄, worin R₄ Wasserstoff oder C₁-C₆-Alkyl bedeutet, steht;
R₂ für Wasserstoff, gegebenenfalls durch Halogen, Hydroxy oder Benzyloxy substituiertes C₁-C₆-Alkyl, C₂-C₆-Alkenyl, C₂-C₆-Alkinyl, -CO₂-C₁-C₆-Alkyl oder -R₅-NR'R'', worin R₅ C₁-C₆-Alkylen, C₂-C₆-Alkenylen oder C₂-C₆-Alkinylen bedeutet und R' und R'' jeweils unabhängig voneinander Wasserstoff oder C₁-C₆-Alkyl bedeuten oder alternativ dazu die Gruppe -NR'R'' als Ganzes 1-Pyrrolidinyl bedeutet, steht und
R₃ für Wasserstoff, Nitro, Amino, Halogen, C₁-C₆-Alkoxy, Hydroxy oder C₁-C₆-Alkyl steht;
oder ein pharmazeutisch unbedenkliches Salz davon zur Verwendung bei der Behandlung von Schizophrenie beim Menschen.

2. Verbindung der Formel I gemäß Anspruch 1 oder ein pharmazeutisch unbedenkliches Salz davon zur Verwendung bei der Behandlung von Schizophrenie beim Menschen unter Vermeidung des begleitenden Risikos von glukoregulatorischen Abnormalitäten, die mit der Verabreichung von Antipsychotika assoziiert sind.

3. Verbindung der Formel I gemäß Anspruch 1 oder ein pharmazeutisch unbedenkliches Salz davon zur Verwendung bei der Behandlung der mit Schizophrenie assoziierten kognitiven Dysfunktion beim Menschen unter Vermeidung des begleitenden Risikos von glukoregulatorischen Abnormalitäten, die mit der Verabreichung von Antipsychotika assoziiert sind.

4. Verbindung der Formel I gemäß Anspruch 1 oder ein pharmazeutisch unbedenkliches Salz davon zur Verwendung bei der Behandlung der mit Schizophrenie assoziierten kognitiven Dysfunktion beim Menschen unter Vermeidung des begleitenden Risikos von mit Schizophrenie in Zusammenhang stehenden Abnormalitäten der Glukoseregulation.

5. Verbindung der Formel I gemäß Anspruch 1 oder ein pharmazeutisch unbedenkliches Salz davon zur Verwendung bei der Behandlung der kognitiven Dysfunktion und glukoregulatorischen Abnormalitäten, die mit Schizophrenie assoziiert sind, beim Menschen.

6. Verbindung der Formel I gemäß Anspruch 1 oder ein pharmazeutisch unbedenkliches Salz davon zur Verwendung bei der Behandlung von glukoregulatorischen Abnormalitäten bei einem Patienten.

7. Verbindung der Formel I gemäß Anspruch 1 oder ein pharmazeutisch unbedenkliches Salz davon zur Verwendung bei der Behandlung der mit Schizophrenie assoziierten negativen Symptome beim Menschen unter Vermeidung des begleitenden Risikos von glukoregulatorischen Abnormalitäten, die mit der Verabreichung von Antipsychotika assoziiert sind.

8. Verbindung der Formel I gemäß Anspruch 1 oder ein pharmazeutisch unbedenkliches Salz davon zur Verwendung bei der Erhöhung der glukosestimulierten Insulinfreisetzung bei einem Patienten.

9. Verbindung zur Verwendung nach einem der Ansprüche 1 bis 8, wobei R für Wasserstoff, Halogen, Trifluormethyl oder C₁-C₆-Alkyl steht; R₁ für Wasserstoff oder C₁-C₆-Alkyl steht; R₂ für Wasserstoff oder C₁-C₆-Alkyl steht; R₃ für Wasserstoff, C₁-C₆-Alkyl oder Halogen steht und p für 0 steht.

10. Verbindung zur Verwendung nach einem der Ansprüche 1 bis 8, wobei die Verbindung die folgende Formel aufweist:

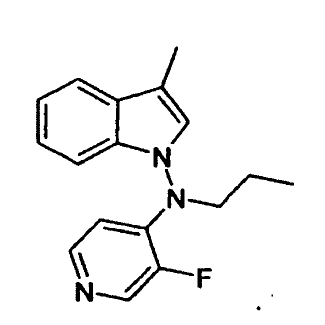

11. Verbindung zur Verwendung nach einem der Ansprüche 1 bis 8, wobei die Verbindung die folgende Formel aufweist:

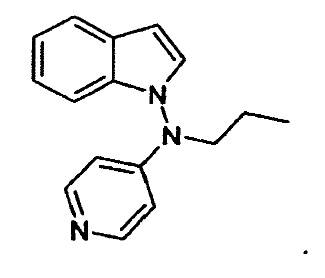

12. Verbindung zur Verwendung nach Anspruch 6, wobei es sich bei der glukoregulatorischen Abnormalität um Hyperglykämie handelt.

13. Verbindung zur Verwendung nach Anspruch 6, wobei es sich bei der glukoregulatorischen Abnormalität um Typ-2-Diabetes mellitus handelt.

14. Produkt, enthaltend (i) eine therapeutisch wirksame Menge einer Verbindung der Formel I gemäß Anspruch 1 oder eines pharmazeutisch unbedenklichen Salzes davon in Kombination mit (ii) einer therapeutisch wirksamen Menge eines Antipsychotikums, zur gleichzeitigen, separaten oder aufeinanderfolgenden Verwendung bei der Behandlung von Schizophrenie beim Menschen unter Vermeidung des begleitenden Risikos von glukoregulatorischen Abnormalitäten, die mit der Verabreichung von Antipsychotika assoziiert sind.

15. Produkt zur Verwendung nach Anspruch 14, wobei R für Wasserstoff, Halogen, Trifluormethyl oder C₁-C₆-Alkyl steht; R₁ für Wasserstoff oder C₁-C₆-Alkyl steht; R₂ für Wasserstoff oder C₁-C₆-Alkyl steht; R₃ für Wasserstoff, C₁-C₆-Alkyl oder Halogen steht und p für 0 steht.

16. Produkt zur Verwendung nach Anspruch 14, wobei die Verbindung die folgende Formel aufweist:

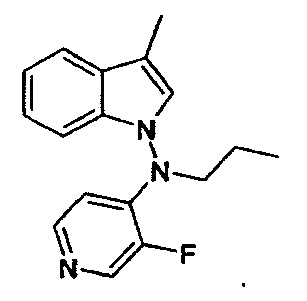

17. Produkt zur Verwendung nach Anspruch 14, wobei die Verbindung die folgende Formel aufweist:

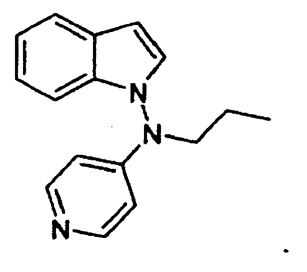

18. Produkt, enthaltend (i) eine therapeutisch wirksame Menge einer Verbindung der Formel I gemäß Anspruch 1 oder eines pharmazeutisch unbedenklichen Salzes davon in Kombination mit (ii) einer therapeutisch wirksamen Menge mindestens eines anderen Wirkstoffs aus der Gruppe bestehend aus:
Antidiabetika, hypoglykämischen Wirkstoffen, HMGCoA-Reduktase-Inhibitoren, Cholesterinresorptionsinhibitoren, PPAR-gamma-Agonisten, PPAR-alpha-Agonisten, PPAR-alpha/gamma-Agonisten, Fibraten, MTP-Inhibitoren, Gallensäureresorptionsinhibitoren, CETP-Inhibitoren, polymeren Gallensäureadsorbern, LDL-Rezeptor-Inducern, ACAT-Inhibitoren, Antioxidantien, Lipoprotein-Lipase-Inhibitoren, ATP-Citrat-Lyase-Inhibitoren, Squalensynthetase-Inhibitoren, Lipoprotein(a)-Antagonisten, Lipase-Inhibitoren, Insulinen, Sulfonylharnstoffen, Biguaniden, Meglitiniden, Thiazolidindionen, α-Glukosidase-Inhibitoren, auf den ATP-abhängigen Kaliumkanal der Betazellen wirkenden Wirkstoffen, CART-Agonisten, NPY-Agonisten, MC4-Agonisten, Orexin-Agonisten, H3-Agonisten, TNF-Agonisten, CRF-Agonisten, CRF-BP-Antagonisten, Urocortin-Agonisten, β3-Agonisten, MSH-Agonisten (MSH = Melanocyt-stimulierendes Hormon), CCK-Agonisten, Serotonin-Wiederaufnahme-Inhibitoren, gemischten sertoninergen und noradrenergen Verbindungen, 5HT-Agonisten, Bombesin-Agonisten, Galanin-Antagonisten, Wachstumshormonen, Wachstumshormon freisetzende Verbindungen, TRH-Agonisten, entkoppelnden Protein-2- oder -3-Modulatoren, Leptin-Agonisten, DA-Agonisten (Bromocriptin, Doprexin), Lipase/Amylase-Inhibitoren, PPAR-Modulatoren, RXR-Modulatoren oder TR-β-Agonisten oder Amphetaminen, zur gleichzeitigen, separaten oder aufeinanderfolgenden Verwendung bei der Behandlung von glukoregulatorischen Abnormalitäten bei einem Patienten.

## Revendications

1. Composé de formule I 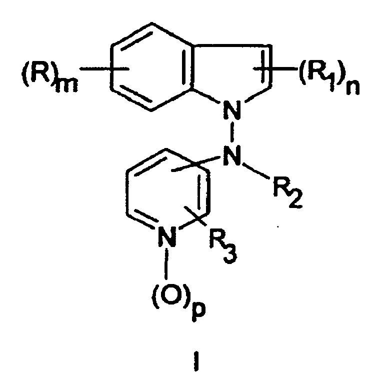 dans laquelle
m est 0, 1 ou 2 ;
n est 0, 1 ou 2 ;
p est 0 ou 1 ;
chaque R est indépendamment hydrogène, halogène, trifluorométhyle, C₁-C₆-alkyle, C₁-C₆-alcoxy, benzyloxy, hydroxy, nitro ou amino ;
chaque R₁ est indépendamment hydrogène, C₁-C₆-alkyle, C₂-C₆-alcényle, C₁-C₆-alcanoyle, halogène, cyano, -C(O)-C₁-C₆-alkyle, -C₁-C₆-alkylène-CN, -C₁-C₆-alkylène-NR'R'', où R' et R'' sont chacun indépendamment hydrogène ou C₁-C₆-alkyle, -C₁-C₆-alkylène-OC(O)-C₁-C₆-alkyle ou -CH(OH)R₄, où R₄ est hydrogène ou C₁-C₆-alkyle ;
R₂ est hydrogène, C₁-C₆-alkyle éventuellement substitué par halogène, hydroxy ou benzyloxy, C₂-C₆-alcényle, C₂-C₆-alcynyle, -CO₂-C₁-C₆-alkyle ou -R₅-NR'R'', où R₅ est C₁-C₆-alkylène, C₂-C₆-alcénylène ou C₂-C₆-alcynylène et R' et R'' sont chacun indépendamment hydrogène ou C₁-C₆-alkyle ou de manière alternative le groupement -NR'R'' en entier est 1-pyrrolidinyle ; et
R₃ est hydrogène, nitro, amino, halogène, C₁-C₆-alcoxy, hydroxy ou C₁-C₆-alkyle ;
ou un sel pharmaceutiquement acceptable de celui-ci,
destiné à être utilisé dans le traitement de la schizophrénie chez l'homme.

2. Composé de formule I selon la revendication 1 ou un sel pharmaceutiquement acceptable de celui-ci, destiné à être utilisé dans le traitement de la schizophrénie chez l'homme tout en évitant l'inconvénient concomitant d'anomalies de la glucorégulation associées à l'administration d'agents antipsychotiques.

3. Composé de formule I tel que défini dans la revendication 1 ou un sel pharmaceutiquement acceptable de celui-ci, destiné à être utilisé dans le traitement du dysfonctionnement cognitif associé à la schizophrénie chez l'homme tout en évitant l'inconvénient concomitant d'anomalies de la glucorégulation associées à l'administration d'agents antipsychotiques.

4. Composé de formule I tel que défini dans la revendication 1 ou un sel pharmaceutiquement acceptable de celui-ci destiné à être utilisé dans le traitement du dysfonctionnement cognitif associé à la schizophrénie chez l'homme tout en évitant l'inconvénient concomitant d'anomalies de la régulation du glucose liées à la schizophrénie.

5. Composé de formule I tel que défini dans la revendication 1 ou un sel pharmaceutiquement acceptable de celui-ci destiné à être utilisé dans le traitement du dysfonctionnement cognitif et des anomalies de la glucorégulation associés à la schizophrénie chez l'homme.

6. Composé de formule I tel que défini dans la revendication 1 ou un sel pharmaceutiquement acceptable de celui-ci, destiné à être utilisé dans le traitement des anomalies de la glucorégulation chez un patient.

7. Composé de formule I selon la revendication 1 ou un sel pharmaceutiquement acceptable de celui-ci destiné à être utilisé dans le traitement des symptômes négatifs associés à la schizophrénie chez l'homme tout en évitant l'inconvénient concomitant des anomalies de la glucorégulation associées à l'administration d'agents antipsychotiques.

8. Composé de formule I tel que défini dans la revendication 1 ou un sel pharmaceutiquement acceptable de celui-ci destiné à être utilisé pour améliorer la libération d'insuline stimulée par le glucose chez un patient.

9. Composé destiné à être utilisé selon l'une des revendications 1 - 8, **caractérisé en ce que** R est hydrogène, halogène, trifluorométhyle ou C₁-C₆-alkyle ; R₁ est hydrogène ou C₁-C₆-alkyle ; R₂ est hydrogène ou C₁-C₆-alkyle ; R₃ est hydrogène, C₁-C₆-alkyle ou halogène ; et p est 0.

10. Composé destiné à être utilisé selon l'une des revendications 1 - 8, **caractérisé en ce que** le composé a la formule suivante :

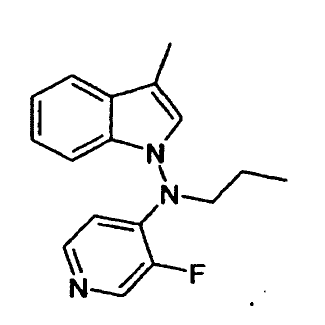

11. Composé destiné à être utilisé selon l'une des revendications 1 - 8, **caractérisé en ce que** le composé a la formule suivants :

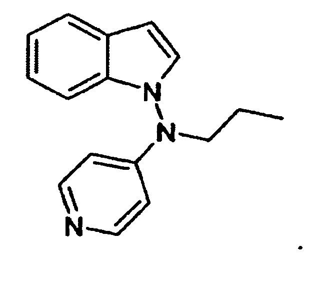

12. Composé destiné à être utilisé selon la revendication 6, **caractérisé en ce que** l'anomalie de la glucorégulation est l'hyperglycémie.

13. Composé destiné à être utilisé selon la revendication 6, **caractérisé en ce que** l'anomalie de la glucorégulation est le diabète sucré de type 2.

14. Produit comprenant (i) une quantité thérapeutiquement efficace d'un composé de formule I tel que défini dans la revendication 1 ou d'un sel pharmaceutiquement acceptable de celui-ci, en combinaison avec (ii) une quantité thérapeutiquement efficace d'un agent antipsychotique destiné à une utilisation simultanée, séparée ou séquentielle dans le traitement de la schizophrénie chez l'homme tout en évitant l'inconvénient concomitant d'anomalies de la glucorégulation associées à l'administration d'agents antipsychotiques.

15. Produit destiné à être utilisé selon la revendication 14, **caractérisé en ce que** R est hydrogène, halogène, trifluorométhyle ou C₁-C₆-alkyle ; R₁ est hydrogène ou C₁-C₆-alkyle ; R₂ est hydrogène ou C₁-C₆-alkyle ; R₃ est hydrogène, C₁-C₆-alkyle ou halogène ; et p est 0.

16. Produit destiné à être utilisé selon la revendication 14, **caractérisé en ce que** le composé a la formule suivante :

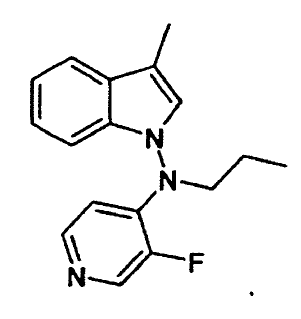

17. Produit destiné à être utilisé selon la revendication 14, **caractérisé en ce que** le composé a la formule suivante :

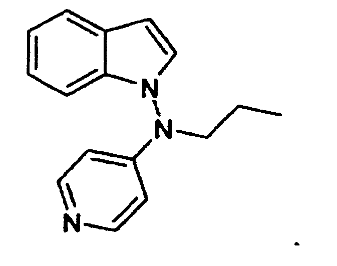

18. Produit comprenant (i) une quantité thérapeutiquement efficace d'un composé de formule I tel que défini dans la revendication 1 ou d'un sel pharmaceutiquement acceptable de celui-ci, en combinaison avec (ii) une quantité thérapeutiquement efficace d'au moins un autre principe actif choisi dans le groupe constitué de ce qui suit :
les agents antidiabétiques, les principes actifs hypoglycémiants, les inhibiteurs de l'HMGCoA réductase, les inhibiteurs de l'absorption du cholestérol, les agonistes du PPAR gamma, les agonistes du PPAR alpha, les agonistes du PPAR alpha/gamma, les fibrates, les inhibiteurs de la MTP, les inhibiteurs de l'absorption de l'acide biliaire, les inhibiteurs de la CETP, les adsorbants polymères de l'acide biliaire, les inducteurs du récepteur de la LDL, les inhibiteurs de l'ACAT, les antioxydants, les inhibiteurs de la lipoprotéine lipase, les inhibiteurs de l'ATP-citrate lyase, les inhibiteurs de la squalène synthétase, les antagonistes de la lipoprotéine(a), les inhibiteurs des lipases, les insulines, les sulfonylurées, les biguanides, les méglitinides, les thiazolidinediones, les inhibiteurs de l'α-glucosidase, les principes actifs agissant sur le canal potassique ATP-dépendant des cellules bêta, les agonistes du CART, les agonistes du NPY, les agonistes du MC4, les agonistes de l'orexine, les agonistes de l'H3, les agonistes du TNF, les agonistes du CRF, les antagonistes de la CRF BP, les agonistes de l'urocortine, les agonistes de β3, les agonistes de la MSH (MSH = hormone de stimulation des mélanocytes), les agonistes de la CCK, les inhibiteurs de la recapture de la sérotonine, les composés sérotoninergiques et noradrénergiques mixtes, les agonistes de la 5HT, les agonistes de la bombésine, les antagonistes de la galanine, les hormones de croissance, les composés de libération de l'hormone de croissance, les agonistes de la TRH, les modulateurs de la protéine découplante 2 ou 3, les agonistes de la leptine, les agonistes de la DA (bromocriptine, dopréxine), les inhibiteurs des lipases/amylases, les modulateurs du PPAR, les modulateurs du RXR ou les agonistes du TR-β ou les amphétamines,
destiné à une utilisation simultanée, séparée ou séquentielle dans le traitement des anomalies de la glucorégulation chez un patient.
